# EUROPEAN PATENT APPLICATION

(11) **EP 3 812 007 A1**
(43) Date of publication of application: **28.04.2021**
(21) Application number: 19205194.4
(22) Date of filing: 24.10.2019
(51) Int. Cl.: A61P 31/04, A61K 31/662, C07F 9/655

(54) **NEW FORMULATIONS OF FOSFOMYCIN WITH REDUCED SODIUM CONTENT FOR PARENTERAL USE AND METHODS OF PRODUCING THE SAME**

(71) Applicant: SANDOZ AG, 4056 Basel (CH)
(72) Inventor: LI, Jun, 6250 Kundl (AT); RANEBURGER, Johannes, 6250 Kundl (AT); IMLER, Josef, 6250 Kundl (AT)
(74) Representative: Prüfer & Partner mbB Patentanwälte · Rechtsanwälte

(57) **Abstract**

The present invention relates to crystalline fosfomycin salts comprising 0.8 to 1.2 equivalents sodium relative to one equivalent fosfomycin, as well as a pharmaceutical composition and injectable preparation comprising the same. The present invention further refers to a method for producing said crystalline fosfomycin salts, pharmaceutical compositions and injectable preparations, and to the use thereof in therapy and in treating a specific patient group.

## Description

### FIELD OF THE INVENTION

The present invention relates to crystalline fosfomycin salts and pharmaceutical compositions comprising crystalline fosfomycin salts as an active pharmaceutical ingredient (API). Furthermore, the present invention relates to methods of producing crystalline fosfomycin salts, as well as to the use of said salts in therapy. The crystalline fosfomycin salts of the present invention and the pharmaceutical compositions comprising the same, such as injectable preparations, as well as the methods of the present invention in particular provide a reduction of undesired cation content.

### BACKGROUND OF THE INVENTION

Fosfomycin, also known as phosphomycin and phosphonomycin, is a broad-spectrum antibiotic that is active against a wide range of gram-positive and gram-negative species, with useful activity against *E. faecalis, E. coli,* and various gram-negative bacteria such as *Citrobacter* and *Proteus.* It is bactericidal and inhibits bacterial cell wall biogenesis by interfering with peptidoglycan biosynthesis. It is a natural compound produced by certain *Streptomyces* species. Nowadays, it is produced by chemical synthesis.

Fosfomycin is clinically indicated in the treatment of urinary tract infections, where it is administered at high doses. Fosfomycin/aminoglycoside combinations have been explored for the treatment of bacterial respiratory infections. Patients suffering from cystic fibrosis were treated with fosfomycin/tobramycin combinations. These combinations were further explored for the treatment of ophthalmic, otological and dermatological infections. Another combination that has been evaluated for its activity against urinary tract infections was fosfomycin/N-acetylcysteine. After more than 40 years of clinical use, intravenous fosfomycin is re-emerging as valuable alternative for the treatment of difficult-to-treat infections such as methicillin-resistant *Staphylococcus aureus,* glycopeptide-resistant *Enterococci* and multidrug-resistant *Enterobacteria.*

Fosfomycin is clinically available as disodium salt (FS.2Na), calcium salt (FS.Ca) or mono-trometamol salt (FS.THAM). The disodium salt is used for intravenous injection, while the calcium and trometamol salt are used for oral administration. The disodium salt (FS.2Na) used for intravenous infusion contains succinic acid (SA) besides FS.2Na, and therefore subsequently is abbreviated FS.2Na/SA. FS.2Na has a very basic pH of above 10, and therefore succinic acid is needed to adjust the pH to a physiological acceptable pH.

The drug is generally well tolerated and has a low incidence of harmful side effects. However, fosfomycin for intravenous use, i.e. the disodium salt, is associated with significant limitations due to high sodium uptake. The maximum daily dosage of fosfomycin disodium is approximately 3 x 10.6 g, corresponding to 3 x 8.0 g fosfomycin and 3 x 6.5 g sodium chloride. Such high amounts of sodium uptake is problematic and should in particular be avoided when treating patients suffering from impaired heart or impaired renal functions or hypernatremia. The clinical use of fosfomycin against multidrug-resistant bacteria is thereby strongly limited. Accordingly, there is a need to provide alternative fosfomycin salt preparations which are better tolerated.

Various methods of producing fosfomycin salts have been reported. US 3 914 231 A describes a method of producing fosfomycin salts from fosfomycin phenylethylamine (FS.PEA) using cation exchange resin and water as solvent. The product FS.PEA was obtained after crystallization. FS.Na and various other FS-salts are also disclosed. Crystalline FS.Na is not disclosed.

EP 0 213 704 A2 describes a method of producing mixtures of the mono- and disodium salt of fosfomycin from fosfomycin phenylethylamine and sodium methoxide using methanol as solvent. The mixed salt contained 65-60% of FS.Na and 40-35% of FS.2Na. The mixture of FS.Na and FS.2Na was precipitated using acetone. Crystal forms are not mentioned.

CN 1539842 A describes a method of producing mixtures of mono- and disodium fosfomycin salts from fosfomycin phenylethylamine and sodium hydroxide using cation exchange resin and water as solvent. The mixed salt contained 65-60% of FS.Na and 40-35% of FS.2Na. Crystal form of the mixed FS.2Na/FS.Na salt is mentioned in the description but no data on crystal forms are disclosed.

GB1239159A (Examples 3, 4, 5) and US3595880A (Examples 2, 4, 6, 8, 9, 10) both describe manufacture methods of monosodium fosfomycin (FS.Na), but the disclosed processes are long and utilize hazardous chemicals (ozone, sodium sulfide, thionyl chloride, diazo ethane, hydrazine, benzene) and employ aggressive reaction conditions (pyrolysis). Crystallization in general terms is mentioned in US3595880A, column 4, line 1-2, but not specifically regarding any specific salt and not specifically regarding FS.Na. No data on any crystal products are disclosed.
Disadvantageously, these methods employ hazardous chemicals and/or hazardous solvents, and/or produce organic waste, which severely complicates the production of fosfomycin salts. In particular if the product is intended to be used as a pharmaceutical, the solvents and/or waste has to be removed from the final product. Further, it is advantageous to employ mild synthesis conditions, because this reduces the costs of the chemical synthesis equipment.

Sodium free salts of Fosfomycin are disclosed in GB2013682A (see page 1, left row, line 11-19). In detail salts of the fosfomycin free acid with the proton of the acid being replaced by lysine, arginine, ornithine cysteine, methionine, glycine, citrulline, trimethylammonium derivatives, as for example choline, carnitine, betaine, etc.
As fosfomycin free acid (FS) is not stable in water and has an extremely low pH value (pH < 1), it is not suitable for intravenous (i.v.) use and is usually an intermediate product in a synthesis process.

CN102579466 discloses two formulations containing fosfomycin free acid and huge amounts of Arginine. If the max. therapeutic fosfomycin dose of 24g FS per day is used, these formulations correspond to 84.6 g or 60.6 g Arginine which is more that the physiologically acceptable daily intravenous dose of Arginine for humans. Consequently no pre-clinical data for these formulations are shown in CN102579466.

New methods would therefore be desirable which at least overcome some of the disadvantages encountered by the prior art methods. In particular, there is a need in the art to provide improved methods of producing fosfomycin salts, specifically methods that are more economically friendly facing the disadvantage of using organic solvents and/or producing organic waste. Another object of the present invention is to provide new fosfomycin salt preparations, preferably wherein these salt preparations are well tolerated. A further object is to provide fosfomycin salt having improved physico-chemical property profile including good flow properties, good stability and/or low hygroscopy.

In particular, if fosfomycin salt preparations are intended for being used i.v., it is noted that fosfomycin free acid (FS) is too acidic and too instable and thus not suitable for an i.v. preparation. Using FS.2Na bears the risk of too high sodium uptake. Hence, not least because of these further disadvantages that are particularly in connection with i.v. administration, it is additionally desirable to provide preparations that are stable in water, that finally exhibit physiological pH, and that significantly reduce the risk of too high sodium uptake.

### SUMMARY OF THE INVENTION

These objects are solved by a crystalline fosfomycin salt of claim 1, a pharmaceutical composition comprising the same, by an aqueous solution obtainable or obtained by combining the crystalline fosfomycin salt with an aqueous solution or water, by an injectable preparation, by a method for the respective production thereof, and by the use of said salts, compositons, or solutions.
The dependent claims and the disclosure provide particular embodiments thereof. Accordingly, the present invention provides the following aspects, subject-matter and preferred embodiments, which, respectively taken alone or in combination, contribute to providing improved technical effects and to solving the afore-mentioned object of the invention:
1. Crystalline fosfomycin salt comprising, preferably consisting of/consisting essentially of, 0.8 to 1.2 equivalents sodium relative to one equivalent fosfomycin, preferably 0.8 to 1.1 equivalents sodium relative to one equivalent fosfomycin, more preferably 0.9 to 1.1 equivalents sodium relative to one equivalent fosfomycin, and most preferably about 1 equivalent sodium relative to one equivalent fosfomycin.
2. The crystalline fosfomycin salt according to 1, being characterized by a pH value of 4.0 to 6.0, preferably 4.5 to 5.5, more preferably 5.0 to 5.5, when reconstituted in water. Fosfomycin monosodium (FS.Na) has a pH value of 5.0 (4.5 - 5.5).
3. The crystalline fosfomycin salt according to 1 or 2, being characterized by a pH value of 5.0 ± 0.2 or 5.3 ± 0.2, when reconstituted in water.
4. The crystalline fosfomycin salt according to any of the preceding embodiments, which is free of bases other than sodium, in particular free of any other organic or inorganic compounds in the crystal lattice except for solvents.
5. The crystalline fosfomycin salt according to any of the preceding embodiments, which is fosfomycin monosodium (FS.Na).
6. The crystalline fosfomycin salt according to 5, wherein the fosfomycin monosodium (FS.Na) is characterized by an XRPD pattern as measured using Cu-Kα1.2 radiation at a wavelength of 0.15419 nm comprising peaks at 2-theta angles (°2θ) of 7.5 ± 0.2, 10.3 ± 0.2 and 11.2 ± 0.2 ("**Form 1**").
7. The crystalline fosfomycin salt according to 5, wherein the fosfomycin monosodium (FS.Na) is characterized by an XRPD pattern as measured using Cu-Kα1.2 radiation at a wavelength of 0.15419 nm comprising peaks at 2-theta angles (°2θ) of 6.5 ± 0.2, 7.5 ± 0.2 and 17.1 ± 0.2 ("**Form 2**").
8. The crystalline fosfomycin salt according to 5, wherein the fosfomycin monosodium (FS.Na) is characterized by an XRPD pattern as measured using Cu-Kα1.2 radiation at a wavelength of 0.15419 nm comprising peaks at 2-theta angles (°2θ) of 7.4 ± 0.2, 11.8 ± 0.2 and 18.2 ± 0.2 (**"Form 3**").
9. The crystalline fosfomycin salt according to 5, wherein the fosfomycin monosodium (FS.Na) is characterized by an XRPD pattern as measured using Cu-Kα1.2 radiation at a wavelength of 0.15419 nm comprising peaks at 2-theta angles (°2θ) of 7.4 ± 0.2, 10.9 ± 0.2 and 17.6 ± 0.2 (**"Form** 4").
10. The crystalline fosfomycin salt according to 5, wherein the fosfomycin monosodium (FS.Na) is characterized by an XRPD pattern as measured using Cu-Kα1.2 radiation at a wavelength of 0.15419 nm comprising peaks at 2-theta angles (°2θ) of 7.1 ± 0.2, 16.6 ± 0.2 and 17.9 ± 0.2 (**"Form 5**").
11. The crystalline fosfomycin salt according to 6 or 7, which is characterized by one or both of the following characteristics:
   - having flow characteristics as determined by the Hausner ratio, wherein said Hausner ratio is 1.26 or less, preferably 1.24 or less, more preferably 1.23 or less;
   - having a hygroscopicity wherein the water uptake at 40 % r.h. is less than 15 %, preferably less than 10 %, more preferably less than 5 % weight increase of the salt due to absorption of water, as determined by dynamic vapor sorption (DVS) at 25°C.
12. Pharmaceutical composition comprising the crystalline fosfomycin salt according to any one of 1 to 11.
13. The pharmaceutical composition according to 12, additionally comprising arginine and/or lysine, wherein the respective amounts of arginine and/or lysine being present in the pharmaceutical composition are such that the pH of the pharmaceutical composition, if reconstituted in water, is in a physiological range of 6.0 to 8.0, and no precipitate is visible after visual examination, for example after eye examination, preferably the pharmaceutical composition consists of/consists essentially of said crystalline fosfomycin salt, arginine and/or lysine.
14. The pharmaceutical composition according to 13, wherein the molar ratio of FS.Na : Arg is in a range of about 1.0 : 0.7-0.95, such as about 1.0 : 0.9 or about 1.0 : 0.8 or about 1.0 : 0.7; and/or wherein
   the molar ratio of FS.Na : Lys is in a range of about 1.0 : 0.35-0.95, such as about 1.0 : 0.8, or about 1.0 : 0.7, or about 1:0 : 0.6, or about 1.0 : 0.5. Preferably the molar ratio of FS.Na : Lys is about 1.0 : 0.6.
   In a further embodiment, the molar ratio of FS.Na : Arg : Lys is in a range of about
   1.0 : 0.1-0.5 : 0.1-0.4; preferably of about
   1.0 : 0.4 : 0.15; preferably of about
   1.0 : 0.2 : 0.15; preferably of about
   1.0 : 0.40 : 0.28; preferably of about
   1.0 : 0.45 : 0.15; more preferably of about
   1.0 : 0.3 : 0.3.
15. The pharmaceutical composition according to 13 or 14, either comprising, in addition to the FS.Na being present, arginine and no lysine; or lysine and no arginine; or arginine and lysine; preferably, the pharmaceutical composition comprises in addition to the FS.Na arginine and no lysine.
16. The pharmaceutical composition according to 13-15, being capable of forming an aqueous solution for parenteral administration, preferably for intravenous (i.v.) injection.
17. The pharmaceutical composition according to 15 or 16, being a solid preparation.
18. The pharmaceutical composition of 17, wherein said pharmaceutical composition is a powder.
19. Pharmaceutical composition which is an Injectable preparation comprising, preferably consisting of/consisting essentially of, a solution of FS.Na, arginine and/or lysine in water, wherein said injectable preparation has a pH in a physiological range of 6.0 to 8.0 and is ready to use, preferably wherein no precipitate is visible after visual examination, for example after eye examination.
20. Aqueous solution obtainable or obtained by combining the crystalline fosfomycin salt of any of 1 to 11 or the pharmaceutical composition of any of 12 to 18 with an aqueous solution or water, preferably water for injection, and optionally further pharmaceutically acceptable excipients, or no further pharmaceutically acceptable excipients.
21. The aqueous solution of 20, wherein the aqueous solution is in the form of an injectable preparation, preferably ready to use, wherein the injectable preparation comprises infusions, and injections using suitable means such as a syringe; preferably, the injectable preparation is an infusion.
22. Injectable preparation comprising, preferably consisting of, the aqueous solution as defined in 20 or 21.
23. Injectable preparation according to 22, being ready to use.
24. The crystalline fosfomycin salt of any one of 1 to 11, the pharmaceutical composition according to any of 12 to 19, the aqueous solution of 20 or 21, or the injectable preparation of 22 or 23, for parenteral use in therapy. Due to the reduced sodium content of the crystalline fosfomycin salt, the crystalline fosfomycin salt, the pharmaceutical composition, the aqueous solution, and the injectable preparation respectively according to the present invention, they are especially suitable for use in therapy of patients which are sensitive against certain cations such sodium, for example a condition for which limitation of uptake of certain cations such as sodium is clinically indicated. In line with this, the crystalline fosfomycin salt, the pharmaceutical composition, the aqueous solution, and the injectable preparation of the present invention are particular suitable and advantageous for use in treating patients, particularly critically ill patients, with comorbid conditions including cardiac insufficiency, hypertension, pulmonary oedema and diabetes. For patients with heart failure, sodium restriction is commonly recommended to prevent fluid retention, exacerbation of symptoms and hospitalization due to heart failure. Control of sodium is also crucial for the management of diabetic and non-diabetic CDK patients for the control of blood pressure. In particular, the crystalline fosfomycin salt, the pharmaceutical composition, the aqueous solution, and the injectable preparation of the present invention are particularly advantageous for parenteral use in therapy of patients suffering from impaired heart function and impaired renal function.
25. The crystalline fosfomycin salt, the pharmaceutical composition, or the aqueous solution, or injectable preparation of 24, wherein the parenteral use in therapy involves treatment of a patient having a bacterial infection.
   In a preferred embodiment, the patients that have the bacterial infection as disclosed herein additionally suffer from the conditions disclosed in item 24 above.
26. The crystalline fosfomycin salt, the pharmaceutical composition, or the aqueous solution, or injectable preparation of 25, wherein the bacterial infection is a genitourinary disease, in particular urinary tract infection.
27. The crystalline fosfomycin salt, the pharmaceutical composition, or the aqueous solution, or injectable preparation of 26, wherein the bacterial infection is selected from the group consisting of Gram positive and Gram negative bacterium infection; MRSA infection; VRSA infection; infections of the respiratory tract such as cystic fibrosis, bacterial pneumonia, in particular Klebsiella pneumoniae infection, lung infection, bronchiectasis, chronic bronchitis, chronic obstructive pulmonary disease, lung inflammation, lung abscess and tracheobronchitis; as well as ophthalmic, otological and dermatological infections such as infections of the skin and of soft tissue, and infections after burns, infections of the urinary tract, infections of the bile ducts, perioperative infections, endocarditis, sepsis, osteomyelitis, infections of the central nervous system (meningitis, encephalitis, brain abscess) and oto-, rhino-, laryngological infections.
28. The crystalline fosfomycin salt, the pharmaceutical composition, or the aqueous solution, or injectable preparation of 25 to 27, wherein the bacterial infection is selected from the group consisting of:
   - Acinetobacter infection, in particular Acinetobacter baumanii infection;
   - Clostridia infection such as Clostridium difficile infection;
   - Pseudomonas infection;
   - Pyelonephritis caused by bacterial infection;
   - Enterobacteriaceae infection such as Escherichia coli infection;
   - Enterococcaceae infection;
   - Enterococcus infection such as Enterococcus faecalis infection;
   - Haemophilus infection, in particular Haemophilus influenzae infection;
   - Neisseria infection, in particular Neisseria gonorrhoeae infection;
   - Proteus infection, in particular Proteus vulgaris infection;
   - Pseudomonas infection, in particular Pseudomonas aeruginosa infection;
   - Staphylococcus infection such as Staphylococcus aureus infection and Staphylococcus hominis infection; and
   - Stenotrophomonas infection, in particular Stenotrophomonas maltophilia infection.
29. Method for producing a crystalline fosfomycin salt comprising 0.5 to 1.2, preferably 0.8 to 1.2, further preferred about 1 (FS.Na), equivalents sodium relative to one equivalent fosfomycin, the method comprising the following steps:
   (a) providing a solution or suspension comprising fosfomycin disodium (FS.2Na) in a solvent;
   (b) neutralizing one equivalent of sodium in said FS.2Na of step (a) with an acid, or with a cation exchange resin (CER);
   (c) combining the solution of step (b), optionally after filtration, with an anti-solvent; and
   (d) recovering said crystallized fosfomycin salt, preferably crystalline fosfomycin monosodium (FS.Na), preferably a crystalline fosfomycin salt according to any one of Forms 1, 2, 3, 4 and 5.
30. The method of 29, wherein
   - the solvent of step (a) is selected from the group consisting of water, alcohol (preferably methanol), dimethyl sulfoxide, or a mixture thereof;
   - the acid of step (b) is selected from sulfuric acid, hydrochloric acid, oxalic acid, trifluoroacetic acid, trichloroacetic acid, formic acid, acetic acid, or a mixture thereof;
   - the cation exchange resin of step (b) is selected from the group consisting of Dowex® (sulfonic acid), Duolite® (sulfonic acid), Dowex® (carboxylic acid), and Amberlite®.
      The anti-solvent is preferably ethanol.
31. The method of 30, wherein the crystalline fosfomycin salt is as defined in any of 1 to 11.
32. The method of any one of 29 to 31, wherein the FS.2Na used in step (a) is synthesized by using FS.PEA or FS.THAM.
33. The method of any one of 29 to 32, wherein the solution prepared in (b) is sterile.
34. The method of 33, wherein the sterile solution is obtained by sterile filtration.
35. Use of crystalline fosfomycin salt of any one of 1 to 11, the pharmaceutical composition of any of 12 to 19, or the aqueous solution of 20 or 21, for the preparation of an injectable preparation of any one of 22 or 23.
36. Use of crystalline fosfomycin salt of any one of 1 to 11 without any addition of arginine or lysine or any other buffering agent for the preparation of an injectable preparation.
37. Injectable preparation comprising, preferably consisting of/consisting essentially of a solution of FS.Na.
38. Method of preparing a formulation comprising FS.Na and arginine and/or lysine, comprising the step of:
   mixing solid FS.Na and solid arginine and/or lysine as a powder into a single container, for example resulting in a molar ratio of a final solid product as disclosed in Table 3.
39. Method of preparing a formulation comprising FS.Na and arginine and/or lysine, comprising the steps of
   (i) placing solid FS.Na in a first container and
   (ii) placing in a second container a stable liquid solution of arginine and/or lysine in a pharmaceutically acceptable solvent such as water is, and
   (iii) providing an instruction leaflet explaining to the user of the product, how to use the arginine and/or lysine solution of the second container to dissolve the solid FS.Na present in the first container, and dissolving the contents of the first container using the liquid of the second container resulting for example in a molar ratio of the final product as disclosed in Table 3.
40. Method of preparing a formulation comprising FS.Na and arginine and/or lysine, comprising the steps of
   (i) preparing a liquid solution of FS.Na and a liquid solution of arginine and/or lysine using a pharmaceutically acceptable solvent such as water. If both arginine and lysine are desired to be present in the final product, it is possible to prepare one solution containing arginine and lysine within the same solution, or it is possible to prepare a separate arginine solution and a separate lysine solution
   (ii) combining the solutions if step (i).
   (iii) removing the solvent of the combined solution of step 2, for example by commonly known methods such as freeze-drying, lyophilization, etc. under conditions such as temperature and pressure, which ensure that FS.Na and arginine and/or lysine are not subject to degradation and the final solid product for example is obtained in a molar ratio as disclosed in Table 3.
41. Method according to 38 to 40, wherein as starting material sterile FS.Na, sterile arginine and/or sterile lysine is used.
42. Method according to 39 or 40, wherein the liquid materials are sterilized by for example sterile filtration.
43. Method according to 38 to 40, wherein the solid starting materials FS.Na and/or arginine and/or lysine are sterilized, for example by radiation, such as gamma-radiation.
44. Method according to 38 to 40, wherein the solid final product is sterilized by radiation, such as gamma-radiation of the finally packaged solid dose form.
45. Pharmaceutical dose form comprising a package comprising two containers, wherein a first container comprises solid FS.Na and a second container comprises a solution of arginine and/or lysine intended to solubilize the solid FS.Na present in the first container, resulting for example in a molar ratio as disclosed in Table 3.

### DEFINITIONS

Unless otherwise indicated or unless the context dictates another meaning, the term "fosfomycin" herein collectively denotes fosfomycin in its various chemical forms including its protonated and partially or fully deprotonated forms.

The term "salt" encompasses, without being limited by theory, all kinds of acid-base association products such as acid-base complexes, regardless of the actual form of protonation on a molecular level in the solid state. The acid-base association product in the context of fosfomycin preferably denotes a product obtained from fosfomycin by addition of a base (B) and preferably held together by opposite charges. Preferably, the term does not encompass the free acid form of fosfomycin which is not associated with a base.

The term "comprises" herein means that other elements may be included in addition to the element(s) explicitly mentioned. In specific embodiments, the term "comprises" has the meaning of " substantially consisting of" or "consisting of", i.e. meaning that (substantially) no further elements are comprised in addition to the element(s) explicitly mentioned.

The term "about" herein denotes a maximum deviation of 5%, preferably a maximum deviation of 2%, from the respectively indicated value.

### DETAILED DESCRIPTION OF THE INVENTION

The invention is described below in further detail by embodiments, without being limited thereto.

In one aspect, the present invention relates to crystalline fosfomycin salt comprising 0.5 to 1.2, preferably 0.8 to 1.2, further preferred about 1 (FS.Na), equivalents sodium relative to one equivalent fosfomycin.
The present invention also relates to crystalline fosfomycin salt comprising, preferably consisting of/consisting essentially of, 0.8 to 1.2 equivalents sodium relative to one equivalent fosfomycin, preferably 0.8 to 1.1 equivalents sodium relative to one equivalent fosfomycin, more preferably 0.9 to 1.1 equivalents sodium relative to one equivalent fosfomycin, and most preferably about 1 equivalent sodium relative to one equivalent fosfomycin.

Hence, the present invention provides sodium content reduced fosfomycin in the form of FS.Na. This sodium reduced fosfomycin provides for multiple advantages, for example it contributes to avoiding health troubles in patients that suffer from diseases that are in connection with too high sodium level, such as patients that suffer from impaired heart or impaired renal functions or hypernatremia.
Preferably, the salt is characterized by a pH value of 4.0 to 6.0, preferably 4.5 to 5.5, more preferably 5.0 to 5.5, in particular 5.0 ± 0.2 and 5.3 ± 0.2 when reconstituted in water. The term "water" in the present context means neat water that is free of other components, in particular free of buffering substances, such as water for injection (WFI). That is, when the fosfomycin salt alone is reconstituted in such water, such as an amount of 9.27 g fosfomycin salt in 200 mL of water, the pH value of the resulting solution will be in a range of, e.g., 4.0 to 6.0, preferably 4.5 to 5.5, most preferably about 5.0.
It is additionally preferred that the crystalline fosfomycin salt is free of bases other than sodium, in particular free of any other organic or inorganic compounds in the crystal lattice except for solvents. The solvents that may be present can for example belong to the group of class 2 solvents and/or class 3 solvents. An example for a solvent belonging to class 2 is methanol; and examples for solvents belonging to class 3 are ethanol and acetone. If solvents are present, their content is below the limit set by competent regulatory authorities. Details about such limits are known by the skilled person and are for example disclosed in the European Pharmacopoeia 7.0, chapter 5.4., page 583-590.
It is particularly preferred that the crystalline fosfomycin salt is fosfomycin monosodium (FS.Na).

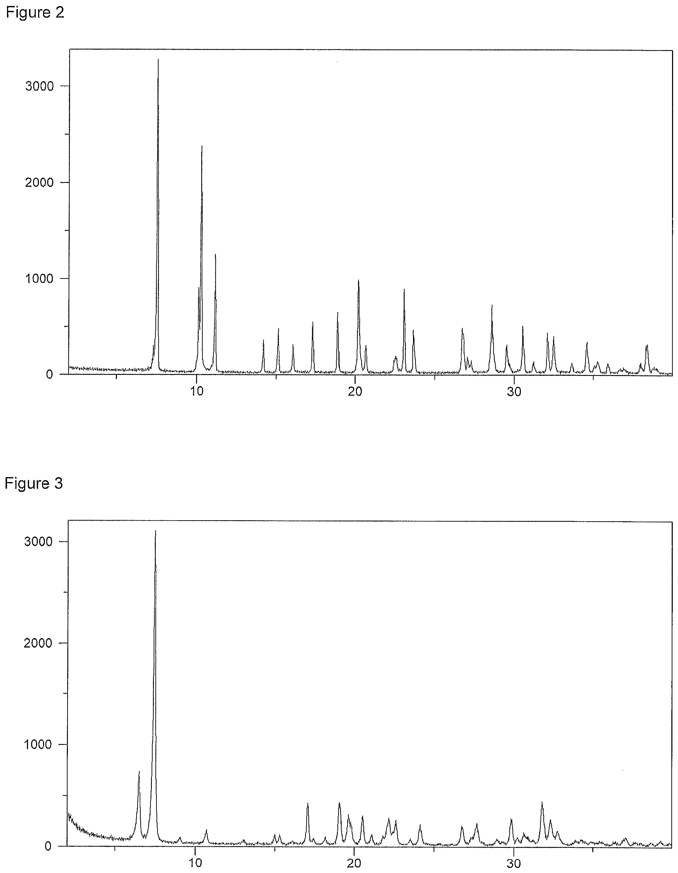

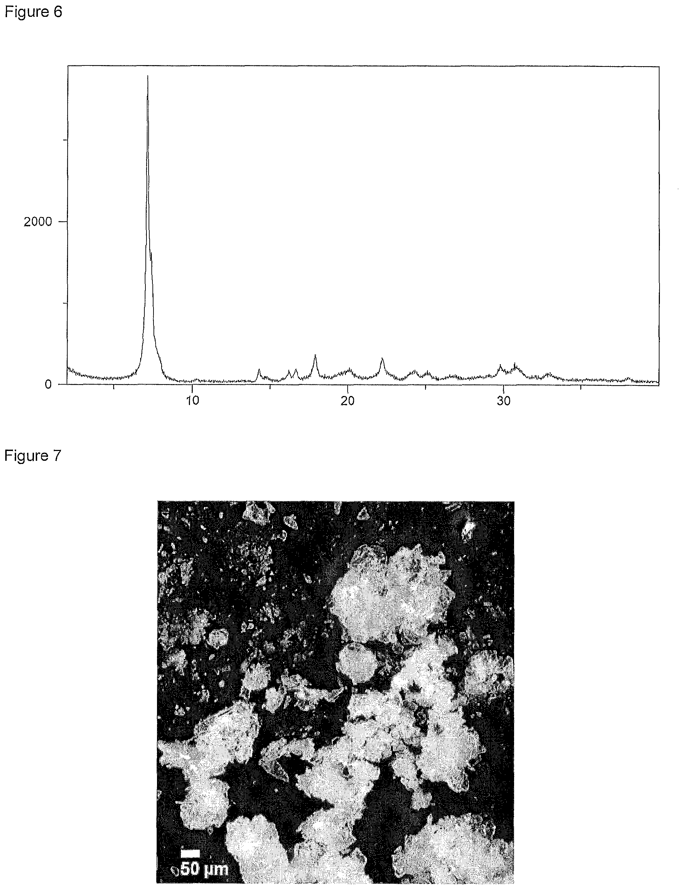

In a preferred embodiment, the crystalline fosfomycin salt is fosfomycin monosodium (FS.Na) being characterized by an XRPD pattern as measured using Cu-Kα1.2 radiation comprising peaks at 2-theta angles (°2θ) of 7.5 ± 0.2, 10.3 ± 0.2 and 11.2 ± 0.2 (herein also referred to as "**Form 1**"). Preferably, the XRPD pattern, in addition to the peaks at 7.5 ± 0.2, 10.3 ± 0.2 and 11.2 ± 0.2, comprises at least one peak at 2-theta angles of 15.1± 0.2 and 17.3± 0.2. Preferably, the XRPD pattern, in addition to the peaks at of 7.5 ± 0.2, 10.3 ± 0.2 and 11.2 ± 0.2, comprises peaks at 2-theta angles of 15.1± 0.2 and 17.3± 0.2, and optionally at least one peak at 2-theta angles of 20.2± 0.2 and/or 23.1± 0.2.
Most preferably, the crystalline FS.Na ("**Form 1**") is characterized by an XRPD pattern as measured using Cu-Kα1.2 radiation comprising peaks at 2-theta angle shown in Example 9,
Table 1, and Figure 2, and/or the crystals of crystalline FS.Na ("**Form 1**") under a microscope look like shown in Figure 7. Relative reflection intensities may optionally be taken into account as qualitative measure (and only as qualitative measure, since intensities can vary due to e.g. crystallinity, preferred orientation, particle size, sample preparation).

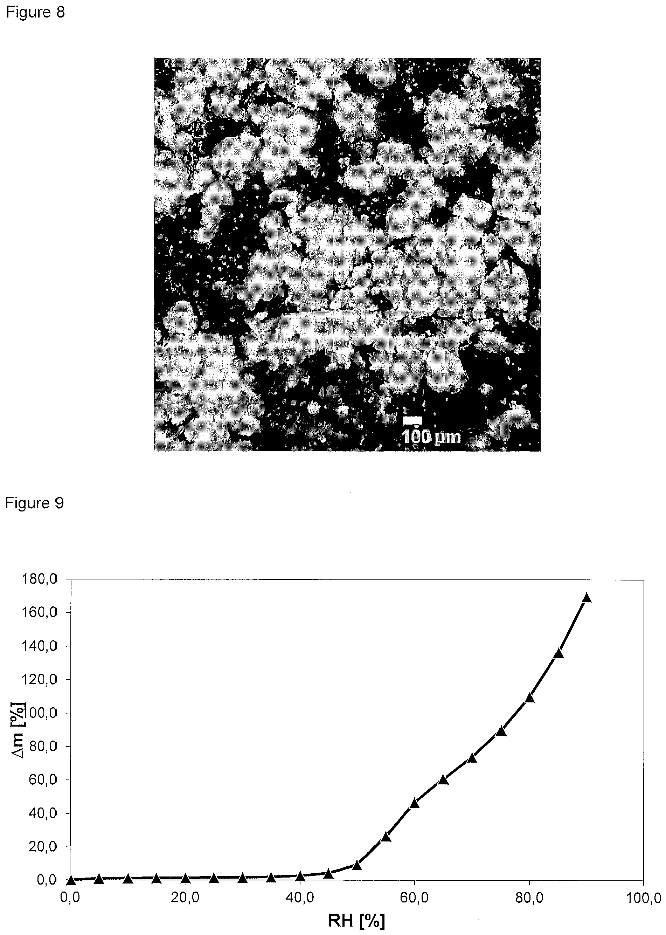

In a further preferred embodiment, the crystalline fosfomycin salt is fosfomycin monosodium (FS.Na) being characterized by an XRPD pattern as measured using Cu-Kα1.2 radiation comprising peaks at 2-theta angles (°2θ) of 6.5 ± 0.2, 7.5 ± 0.2 and 17.1 ± 0.2 (herein also referred to as "**Form 2**"). Preferably, the XRPD pattern, in addition to the peaks at 6.5 ± 0.2, 7.5 ± 0.2 and 17.1 ± 0.2, comprises at least one peak at 2-theta angles of 19.1± 0.2 and 19.6± 0.2. Preferably, the XRPD pattern, in addition to the peaks at of 6.5 ± 0.2, 7.5 ± 0.2 and 17.1 ± 0.2, comprises peaks at 2-theta angles of 19.1± 0.2 and 19.6± 0.2, and optionally at least one peak at 2-theta angles of 29.8± 0.2 and/or 31.8± 0.2.
Most preferably, the crystalline FS.Na ("**Form 2**") is characterized by an XRPD pattern as measured using Cu-Kα1.2 radiation comprising peaks at 2-theta angle shown in Example 9, Table 1, and Figure 3, and/or the crystals of crystalline FS.Na ("**Form 2**") under a microscope look like shown in Figure 8. Relative reflection intensities may optionally be taken into account as qualitative measure (and only as qualitative measure, since intensities can vary due to e.g. crystallinity, preferred orientation, particle size, sample preparation).

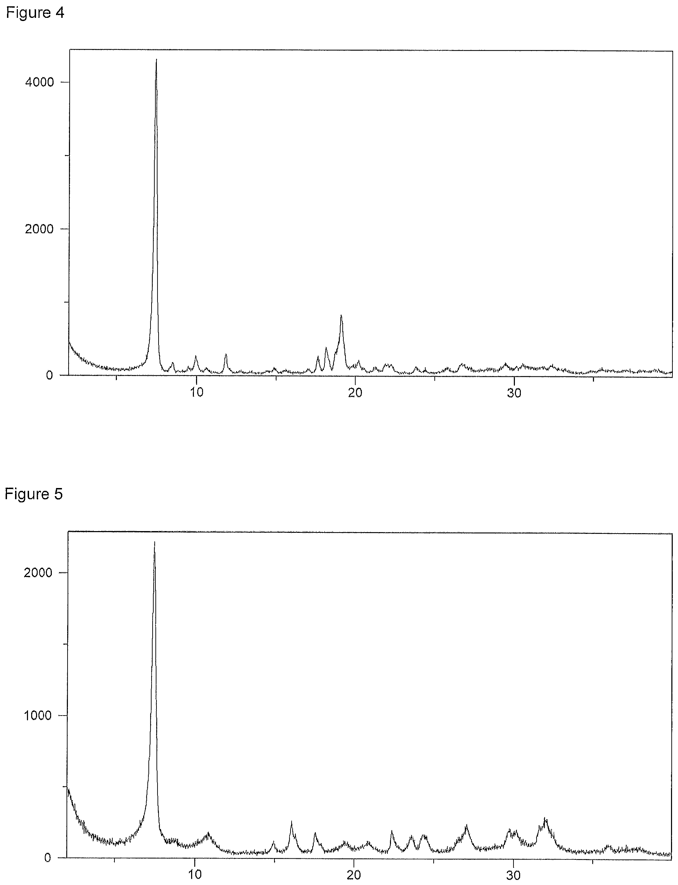

In a further preferred embodiment, the crystalline fosfomycin salt is fosfomycin monosodium (FS.Na) being characterized by an XRPD pattern as measured using Cu-Kα1.2 radiation comprising peaks at 2-theta angles (°2θ) of 7.4 ± 0.2, 11.8 ± 0.2 and 18.2 ± 0.2 (herein also referred to as "**Form 3**"). Preferably, the XRPD pattern, in addition to the peaks at 7.4 ± 0.2, 11.8 ± 0.2 and 18.2 ± 0.2, comprises at least one peak at 2-theta angles of 17.6± 0.2 and 19.1± 0.2. Preferably, the XRPD pattern, in addition to the peaks at of 7.4 ± 0.2, 11.8 ± 0.2 and 18.2 ± 0.2, comprises peaks at 2-theta angles of 17.6± 0.2 and 19.1± 0.2 and optionally at least one peak at 2-theta angles of 8.5± 0.2 and/or 9.9± 0.2.
Most preferably, the crystalline FS.Na ("**Form 3**") is characterized by an XRPD pattern as measured using Cu-Kα1.2 radiation comprising peaks at 2-theta angle shown in Example 9, Table 1, and Figure 4. Relative reflection intensities may optionally be taken into account as qualitative measure (and only as qualitative measure, since intensities can vary due to e.g. crystallinity, preferred orientation, particle size, sample preparation).

In a further preferred embodiment, the crystalline fosfomycin salt is fosfomycin monosodium (FS.Na) being characterized by an XRPD pattern as measured using Cu-Kα1.2 radiation comprising peaks at 2-theta angles (°2θ) of 7.4 ± 0.2, 10.9 ± 0.2 and 17.6 ± 0.2 (herein also referred to as **"Form 4**"). Preferably, the XRPD pattern, in addition to the peaks at 7.4 ± 0.2, 10.9 ± 0.2 and 17.6 ± 0.2, comprises at least one peak at 2-theta angles of 16.1± 0.2 and 22.4± 0.2. Preferably, the XRPD pattern, in addition to the peaks at of 7.4 ± 0.2, 10.9 ± 0.2 and 17.6 ± 0.2, comprises peaks at 2-theta angles of 16.1± 0.2 and 22.4± 0.2 and optionally at least one peak at 2-theta angles of 27.1± 0.2 and/or 32.0± 0.2.
Most preferably, the crystalline FS.Na ("**Form 4**") is characterized by an XRPD pattern as measured using Cu-Kα1.2 radiation comprising peaks at 2-theta angle shown in Example 9, Table 1, and Figure 5. Relative reflection intensities may optionally be taken into account as qualitative measure (and only as qualitative measure, since intensities can vary due to e.g. crystallinity, preferred orientation, particle size, sample preparation).

In a further preferred embodiment, the crystalline fosfomycin salt is fosfomycin monosodium (FS.Na) being characterized by an XRPD pattern as measured using Cu-Kα1.2 radiation comprising peaks at 2-theta angles (°2θ) of 7.1 ± 0.2, 16.6 ± 0.2 and 17.9 ± 0.2 (herein also referred to as **"Form 5**"). Preferably, the XRPD pattern, in addition to the peaks at 7.1 ± 0.2, 16.6 ± 0.2 and 17.9 ± 0.2, comprises at least one peak at 2-theta angles of 14.3± 0.2 and 22.2± 0.2. Preferably, the XRPD pattern, in addition to the peaks at of 7.1 ± 0.2, 16.6 ± 0.2 and 17.9 ± 0.2, comprises peaks at 2-theta angles of 14.3± 0.2 and 22.2± 0.2 and optionally at least one peak at 2-theta angles of 29.8± 0.2 and/or 30.8± 0.2.
Most preferably, the crystalline FS.Na ("**Form 5**") is characterized by an XRPD pattern as measured using Cu-Kα1.2 radiation comprising peaks at 2-theta angle shown in Example 9, Table 1, and Figure 6. Relative reflection intensities may optionally be taken into account as qualitative measure (and only as qualitative measure, since intensities can vary due to e.g. crystallinity, preferred orientation, particle size, sample preparation).

X-ray powder diffraction patterns (XRPD) are obtainable with a PANalytical X'Pert PRO diffractometer equipped with a theta/theta coupled goniometer in transmission geometry, Cu-Kα1.2 radiation (wavelength 0.15419 nm) with a focusing mirror and a solid state PIXcel detector. The patterns may be recorded at a tube voltage of 45 kV and a tube current of 40 mA, applying a 2-theta step size of 0.013° with 40 s per step (255 channels) in the 2-theta angular range of 2° to 40° at ambient conditions.

The reflection peaks (reflection positions) in an X-ray diffractogram are caused at certain diffraction angles (Bragg angles) by constructive interference from X-rays scattered by parallel planes of atoms in solid material, which are distributed in an ordered and repetitive pattern in a long-range positional order. Such a solid material is classified as crystalline material, whereas amorphous material is defined as solid material, which lacks long-range order and only displays short-range order, thus resulting in broad scattering. According to literature, long-range order e.g. extends over approximately 100 to 1000 atoms and more, whereas short-range order is over a few atoms only (see *"*Fundamentals of Powder Diffraction and Structural Characterization of Materials" by Vitalij K. Pecharsky and Peter Y. Zavalij, Kluwer Academic Publishers, 2003, page 3). Deviations from reflection / peak positions and relative intensities are to be taken into account. For example, a typical precision of the 2-theta values is in the range of ± 0.2° 2-theta, preferably in the range of ± 0.1° 2-theta. Thus, a peak/reflection that usually appears at 5.6° 2-theta for example can appear between 5.4° and 5.8° 2-theta, preferably between 5.5 and 5.7° 2-theta on most X-ray diffractometers under standard conditions. Furthermore, one skilled in the art will appreciate that relative peak/reflection intensities will show inter-apparatus variability as well as variability due to degree of crystallinity, preferred orientation, sample preparation and other factors known to those skilled in the art and should be taken as qualitative measure only.

In accordance with the foregoing, whenever reference is made herein to a particular 2-theta angle, the indicated value of said angle is to be understood to also comprise a deviation of ± 0.2°2-theta (°2θ), preferably a deviation of ± 0.1°2θ, from the indicated value.

Advantageously, the crystalline FS.Na can be produced at large scale. The salt is stable in solid state and readily soluble in water at room temperature. Moreover, the crystalline FS.Na, in particular **Form 1** and **Form 2** (see also elsewhere herein), has a hygroscopicity, wherein the water uptake at 40 % r.h. is less than 15 %, preferably less than 10 %, more preferably less than 5 % weight increase of the salt due to absorption of water, as determined by dynamic vapor sorption (DVS) at 25°C (r.h. = RH = relative humidity).

Another advantage is that the crystalline FS.Na, in particular **Form 1** and **Form 2,** has (have) flow properties that are still acceptable ("passable" and "fair") as determined by Hausner ratio. The Hausner ratio can be determined by the method described in the experimental section. Hence, in a preferred embodiment, the crystalline FS.Na is characterized by one or both of the following characteristics: (a) having flow characteristics as determined by the Hausner ratio, wherein said Hausner ratio is 1.26 or less, preferably 1.24 or less, more preferably 1.23 or less; (b) having a hygroscopicity wherein the water uptake at 40 % r.h. is less than 15 %, preferably less than 10 %, more preferably less than 5 % weight increase of the salt due to absorption of water, as determined by dynamic vapor sorption (DVS) at 25°C.

The invention also relates to a pharmaceutical composition comprising the crystalline fosfomycin salt according to the invention. The pharmaceutical composition may additionally comprise arginine and/or lysine, wherein the respective amounts of arginine and/or lysine being present in the pharmaceutical composition are such that the pH of the pharmaceutical composition, if reconstituted in water, is in a physiological range of 6.0 to 8.0, and no precipitate is visible after visual examination, for example after eye examination, preferably the pharmaceutical composition consists of/consists essentially of said crystalline fosfomycin salt, arginine and/or lysine. In one embodiment, the composition consists or essentially consists of said crystalline fosfomycin salt and arginine and/or lysine in solid form or consists or essentially consists of said fosfomycin salt and arginine and/or lysine in dissolved form in water.

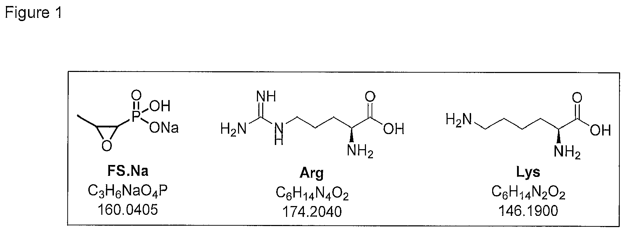

Fig. 1 shows Fosfomycin sodium, Arginine (Arg) and Lysine (Lys).

The molar ratio of FS.Na : Arg can be in a range of about 1.0 : 0.7-0.95, such as 1.0 : 0.7, more preferably of about 1.0 : 0.9, and most preferably of about 1.0 : 0.8.
The molar ratio of FS.Na : Lys can be in a range of about 1.0 : 0.9; preferably of about 1.0 : 0.8, preferably of about 1.0 : 0.7, more preferably about 1:0 : 0.5, most preferably about 1.0 : 0.6.

Thus, in one embodiment, the pharmaceutical composition comprises, in addition to the FS.Na being present, arginine and no lysine; or lysine and no arginine; or arginine and lysine. In a preferred embodiment, the pharmaceutical composition comprises in addition to the FS.Na arginine and no lysine. In the most preferred embodiment, the pharmaceutical composition comprises FS.Na and in addition arginine and lysine.

The pharmaceutical composition is preferably capable of forming an aqueous solution for parenteral administration, preferably for intravenous (i.v.) injection or infusion. The pharmaceutical composition can be a powder.

The invention also relates to an injectable preparation comprising, preferably consisting of/consisting essentially of, a solution of FS.Na, arginine and/or lysine in water, wherein said injectable preparation has a pH in a physiological range of 6.0 to 8.0 and is ready to use, preferably wherein no precipitate is visible after visual examination, for example after eye examination.

The invention also relates to an aqueous solution obtainable or obtained by combining the crystalline fosfomycin salt of the invention or the pharmaceutical composition of any of 12 to 18 with an aqueous solution or water, preferably water for injection, and optionally further pharmaceutically acceptable excipients, or no further pharmaceutically acceptable excipients. The aqueous solution may be in the form of an injectable preparation, preferably ready to use, or ready to dilute with an infusion solution.

The invention also relates to an injectable preparation comprising, preferably consisting of, the aqueous solution of the invention, which is preferably ready to use, or ready to dilute with an infusion solution.

The pharmaceutical composition/injectable preparation of the invention is suitable and/or intended for being administered by injection or infusion. It is further preferred that the preparation is a solid preparation that can be reconstituted (e.g. dissolved) in neat water such as WFI. In preferred embodiments, the pharmaceutical composition/injectable preparation of the invention is sterile, preferably is substantially free of microbes.

The crystalline fosfomycin salt of the invention, the pharmaceutical composition of the invention, the aqueous solution of the invention or the injectable or infusionable preparation of the invention, can be used parenteral in therapy. The pharmaceutical composition/injectable preparation or infusion of the invention is preferably administered by parenteral routes of administration such as injection or infusion by means of a needle or catheter, most preferably intravenous administration.

For stability reasons it is preferred to store the crystalline fosfomycin salt preparation of the invention in dry form and reconstitute it in a suitable solvent (e.g. WFI) before use, preferably by parenteral administration. Said preparation can be stored for up to 24 h at 4°C or RT prior to use, preferably said preparation is prepared immediately prior to use.

The pharmaceutical composition/injectable preparation of the invention can be used as a medicament, either alone or in combination with further active ingredient, preferably further antibiotics. Suitable active ingredients that can be combined with fosfomycin in the crystalline fosfomycin salt preparation of the invention among others are selected from the group consisting of aminoglycosides such as tobramycin, amikacin, imidazole, derivatives of imidazole and N-acetylcysteine. In vitro studies show that a combination of fosfomycin with a-lactam antibiotics, e.g. penicillin, ampicillin, cefazolin, carbapeneme, usually results in an additive to synergistic effect. The same applies to the combination with staphylococcal active substances (linezolid, quinupristin/dalfopristin, moxifloxacin) compared to staphylococcus. With aminoglycoside antibiotics, e.g. gentamicin, fosfomycin usually shows indifferent to additive effects.

The parenteral use in therapy may involve treatment of a patient having a bacterial infection. The bacterial infection is a genitourinary disease, in particular urinary tract infection. The bacterial infection is preferably selected from the group consisting of Gram positive and Gram negative bacterium infection; MRSA infection; VRSA infection; infections of the respiratory tract such as cystic fibrosis, bacterial pneumonia, in particular Klebsiella pneumoniae infection, lung infection, bronchiectasis, chronic bronchitis, chronic obstructive pulmonary disease, lung inflammation and tracheobronchitis; as well as ophthalmic, otological and dermatological infections.

More specifically, diseases that can be treated with the pharmaceutical composition/injectable preparation of the invention are selected from the group consisting of:
- Acinetobacter infection, in particular Acinetobacter baumanii infection;
- Clostridia infection such as Clostridium difficile infection;
- Pseudomonas infection;
- Pyelonephritis caused by bacterial infection;
- Enterobacteriaceae infection such as Escherichia coli infection;
- Enterococcaceae infection;
- Enterococcus infection such as Enterococcus faecalis infection;
- Haemophilus infection, in particular Haemophilus influenzae infection;
- Neisseria infection, in particular Neisseria gonorrhoeae infection;
- Proteus infection, in particular Proteus vulgaris infection;
- Pseudomonas infection, in particular Pseudomonas aeruginosa infection;
- Staphylococcus infection such as Staphylococcus aureus infection and Staphylococcus hominis infection; and
- Stenotrophomonas infection, in particular Stenotrophomonas maltophilia infection.

Most valuable is the present invention for patients in need of a fosfomycin treatment which are sensitive against certain cations such sodium, for example a condition for which limitation of uptake of certain cations such as sodium is clinically indicated. These conditions in particular include impaired heart function and impaired renal function. Therefore, the crystalline fosfomycin salt preparation of the invention is particularly useful for treating patients which are sensitive against certain cations such as sodium, preferably having impaired heart function and/or impaired renal function and/or another condition for which limitation of sodium uptake is clinically indicated. Hence, the low sodium formulation of the present invention is also particularly beneficial and advantageous for critically ill patients with comorbid conditions including cardiac insufficiency, hypertension, pulmonary oedema and diabetes. For patients with heart failure, sodium restriction is commonly recommend to prevent fluid retention, exacerbation of symptoms and hospitalization due to heart failure. Control of sodium is also crucial for the management of diabetic and non-diabetic chronic kidney disease (CDK) patients for the control of blood pressure. The availability of low-sodium formulations would make Fosfomycin available for these critically ill patients and would provide freedom to physicians to prescribe this drug to combat critical cases of AMR (antimicrobial resistance).

The pharmaceutical composition/injectable preparation of the invention is preferably stored in dry (solid) form. It can be either directly administered in solid form or reconstituted to provide a liquid to be administered. The present invention also envisage storage in liquid form, wherein the fosfomycin salt remains as a solid. The invention furthermore envisions a formulation provided in kit comprising two separate containers, one container comprising solid FS.Na and a second container comprising a liquid solution, preferably an aqueous solution comprising arginine and/or lysine.

The invention also refers to the use of crystalline fosfomycin salt of the invention, the pharmaceutical composition of the invention, or the aqueous solution of the invention, for the preparation of an injectable preparation of the invention.

The invention also relates to a method for producing a crystalline fosfomycin salt comprising equivalents sodium relative to one equivalent fosfomycin as indicated elsewhere herein, such as 0.5 to 1.2, or 0.5 to 1.1, preferably 0.8 to 1.2, further preferred about 1 equivalents sodium relative to one equivalent fosfomycin (FS.Na), the method comprising the following steps:
(a) providing a solution or suspension comprising fosfomycin disodium (FS.2Na) in a solvent;
(b) neutralizing one equivalent of sodium in said FS.2Na of step (a) with an acid, or with a cation exchange resin (CER);
(c) combining the solution of step (b), optionally after filtration, with an anti-solvent; and
(d) recovering said crystallized fosfomycin salt, preferably crystalline fosfomycin monosodium (FS.Na), preferably a crystalline fosfomycin salt according to any one of Forms 1, 2, 3, 4 and 5, or mixtures thereof.

The solution in step (a) may be prepared by dissolving/stirring the fosfomycin disodium (FS.2Na) and a hydron source (preferably an acid) in a solvent, which may be selected from alcohol, water, and dimethylsulfoxid (DMSO). Methanol is a particularly useful alcohol. Preparation of the mixture in step (a) may involve stirring. Steps (a)-(c) may be carried out below 25°C. Step (c) is preferably carried out above 10°C and/or below 40°C, preferably at 25°C ± 5 °C.

The solution prepared in step (b) may be filtered, preferably by means of a filter having a nominal pore size of 0.2 to 0.45 µm, in particular 0.2 µm.
Advantageously, in step (c) a first portion of the anti-solvent is combined with the solution and the optional seed crystals simultaneously, and then a second portion of the anti-solvent is added. Step (c) may involve stirring. Optionally, the mixture is further stirred until crystals of fosfomycin monosodium are or have been formed.

In preferred embodiments of the method, the following applies:
- the solvent of step (a) is selected from the group consisting of water, alcohol (preferably methanol), dimethylsulfoxide, or a mixture thereof;
- the acid of step (b) is selected from sulfuric acid, hydrochloric acid, oxalic acid, trifluoroacetic acid, trichloroacetic acid, formic acid, acetic acid, or a mixture thereof;
- the cation exchange resin of step (b) is selected from the group consisting of Dowex® (sulfonic acid), Duolite® (sulfonic acid), Dowex® (carboxylic acid), and Amberlite®.
- the anti-solvent of step (c) may be selected from ethanol, iso-propanol, propanol, butanol, iso-butanol, acetone and MTBE (methyl-tert-butyl-ether). A particularly useful anti-solvent is ethanol. The anti-solvent is preferably ethanol. The crystalline fosfomycin salt obtained by the method is as defined herein, i.e. is the crystalline fosfomycin salt of the invention.

In one embodiment the starting material of step (a), namely FS.2Na, is synthesized by using as a precursor-material FS.PEA or FS.THAM and the resulting FS.2Na is then converted in steps (a) to (d) into FS.Na, as described in the method above.

Preferably, the solution prepared in (b) is sterile. The sterile solution may be obtained by sterile filtration.

As is usual in the field, seed crystals may be added to aid crystallization. The seed crystals comprise crystalline fosfomycin monosodium, which can be produced by the same method, as described above, but without the use of seed crystals.

In the following, an example of a general method for producing a crystalline fosfomycin salt according to the following invention is given:

### 1. Production of FS.Na

### 1.1 Acidification and crystallization process

### a) FS.2Na as starting material

In this process, one equivalent of Na in FS.2Na was neutralized with an acid such as sulfuric acid, hydrochloric acid, oxalic acid, trifluoroacetic acid, trichloroacetic acid, formic acid, acetic acid etc. in a solvent such as water, alcohol, dimethyl sulfoxide or a mixture thereof under pH and temperature control to give FS.Na after crystallization with an anti-solvent such as alcohol, acetone, acetonitrile ect.

### b) FS.PEA as starting material

b)-1) In this process, one equivalent Na in form of a base such as NaOH, sodium alcoholate (MeONa, EtONa etc.) was added to FS.PEA in a solvent such as water, alcohol, dimethyl sulfoxide or a mixture thereof under pH and temperature control to give FS.Na after crystallization with an anti-solvent such as alcohol, acetone, acetonitrile ect.
b)-2) In this process, two equivalents Na in form of a base such as NaOH, sodium alcoholate (MeONa, EtONa etc.) was added to FS.PEA in a solvent such as water, alcohol, dimethyl sulfoxide or a mixture thereof under pH and temperature control to give FS.2Na, which was separated from PEA. In situ, one equivalent of Na was neutralized with an acid such as sulfuric acid, hydrochloric acid, oxalic acid, trifluoroacetic acid, trichloroacetic acid, formic acid, acetic acid etc. in a solvent such as water, alcohol, dimethyl sulfoxide or a mixture thereof under pH and temperature control to give FS.Na after crystallization with an anti-solvent such as alcohol, acetone, acetonitrile ect.

### 1.2 Ion exchange and crystallization process

### c) FS.2Na as starting material

In this process, one equivalent of Na in FS.2Na was neutralized with a cation exchange risin such as Dowex® (sulfonic acid), Duolite® (sulfonic acid), Dowex® (carboxylic acid), Amberlite® etc. in a solvent such as water, alcohol or a mixture thereof under pH and temperature control to give FS.Na after crystallization with an anti-solvent such as alcohol, acetone, acetonitrile ect.

### d) FS.PEA/FS.THAM as starting material

In this process, under pH and temperature control PEA or THAM in FS.PEA or FS.THAM was exchanged by protons using a cation exchange resin in the presence of a solvent such as water, alcohol or a mixture thereof to form fosfomycin free acid, which was neutralized with one equivalent of a base such as NaOH, sodium alcoholate (MeONa, EtONa etc.) under pH and temperature control to give FS.Na after crystallization with an anti-solvent such as alcohol, acetone, acetonitrile ect.

After different crystallization processes, e.g. as described in the examples part, crystal forms of FS.Na were obtained.

### BRIEF DESCRIPTION OF DRAWING

- Figure 1:: Fosfomycin monosodium (FS.Na), Arginine (Arg), Lysine (Lys).
- Figure 2:: XRPD diffractogram of FS.Na-form 1.
- Figure 3:: XRPD diffractogram of FS.Na-form 2.
- Figure 4:: XRPD diffractogram of FS.Na-form 3.
- Figure 5:: XRPD diffractogram of FS.Na-form 4.
- Figure 6:: XRPD diffractogram of FS.Na-form 5.
- Figure 7:: Microscopic picture of FS.Na-form 1 at 200x magnification. 50µm scale bar is shown in the lower right corner.
- Figure 8:: Microscopic picture of FS.Na-form 2 at 100x magnification. 100µm scale bar is shown in the lower right corner.
- Figure 9:: Equilibrium sorption curve of FS.Na-form 1.
- Figure 10:: Equilibrium sorption curve of FS.Na-form 2.
- Figure 11:: Equilibrium sorption curve of FS.2Na (API).
- Figure 12:: Equilibrium sorption curve of FS.2Na/SA (FDF, Astro Pharma).
- Figure 13:: Acidification and crystallization process using FS.2Na.
- Figure 14:: Ion exchange and crystallization process using FS.2Na
- Figure 15:: Ion exchange and crystallization process using FS.THAM

### EXAMPLES

The following abbreviations are used herein:

| | |
|---|---|
| AMR | antimicrobial resistance |
| Arg | Arginine |
| CDK | chronic kidney disease |
| CER | cation exchange resin |
| CF₃CO₂H | Trifluoroacetic acid |
| DVS | Dynamic vapor sorption |
| EtOH | Ethanol |
| EtONa | Sodium ethoxide |
| FS | Fosfomycin or fosfomycin free acid |
| FS.Arg | Fosfomycin arginine |
| FS.2Arg | Fosfomycin diarginine |
| FS.Na | Forsfomycin monosodium |
| FS.2Na | Fosfomycin disodium |
| FS.PEA | Fosfomycin phenylethylamine |
| FS.THAM | Fosfomycin trometamol |
| h | hour |
| HCO₂H | Formic acid |
| H₂O | water |
| H₂SO₄ | Sulfuric acid |
| Lys | Lysine |
| MeOH | Methanol |
| MeONa | Sodium methoxide |
| MRSA | Methicillin-resistant *Staphylococcus aureus* |
| N₂ | Nitrogen gas |
| Na | Sodium |
| NaOH | Sodium hydroxide |
| Na₂SO₄ | Sodium sulfate |
| n.d. | not determined |
| PEA | Phenylethylamine |
| RH / r.h. | Relative humidity |
| RSO₃H | Ion exchange resin |
| SA | Succinic acid |
| THAM | Trometamol |
| VRSA | Vancomycin-Resistant Staphylococcus Aureus |
| WFI | Water for injection |
| XRPD | X-ray powder diffraction patterns |

### Example 1

**Acidification with H₂SO₄ in H₂O and crystallization process using FS.2Na [XRPD: FS.Na-form 1]:** To a suspension of FS.2Na (300 g) in MeOH (1500 mL) were added H₂O (112.5 mL) and a solution of H₂SO₄ in H₂O (50 weight-%, 120 mL) under stirring with temperature (< 22°C) and pH (∼6.8) control. To the resulting solution was added anhydrous sodium sulfate (900 g) under stirring at room temperature. The suspension was filtered under reduced pressure. The cake was washed with MeOH (3 x 150 mL). The pH value of the total filtrate (pH 5.7 at 20°C) was adjusted to 6.06 with a solution of NaOH in H₂O (50 weight-%, 2.15 g). After filtrating through 0.2µm filter, the clear filtrate was divided into three equal portions, to each was added EtOH (1.4 L) under stirring at 35°C in a period of 3h respectively. The resulting mixture was stirred for 1h at 15°C and filtrated. The total precipitation was washed with EtOH (3 x 300 mL), filtrated and dried using nitrogen gas at 35°C in fluid bed dryer for 16h (N₂, 35°C) to give FS.Na (152 g, 57%).

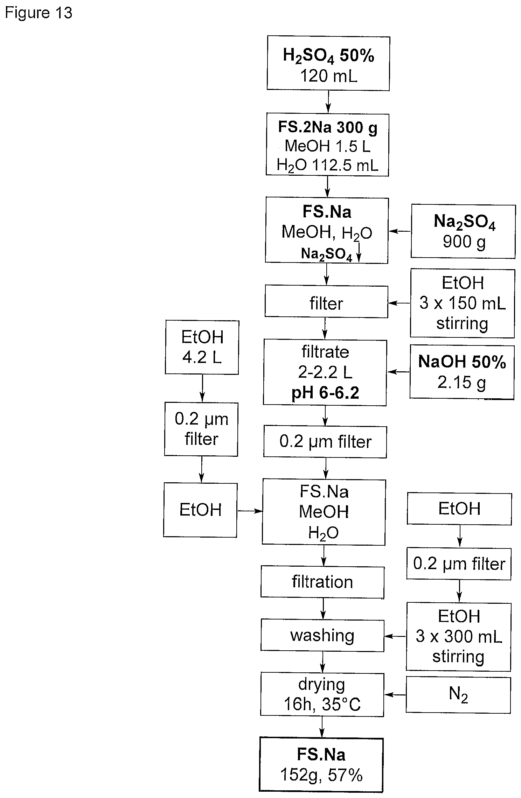

The acidification and crystallization process using FS.2Na is depicted in Fig. 13.

### Example 2

**Ion exchange and crystallization process using FS.2Na [XRPD: FS.Na-form 1]:** To a suspension of FS.2Na (106 g) in MeOH (500 mL) was added ion exchange resin (Dowex Monosphere 650C, 250 g) under stirring with temperature (< 25°C) and pH (∼6.1) control. The mixture was stirred at room temperature for equilibrium of pH (∼6.1) and then filtrated (0.2µm). To the filtrate (pH 6.1 at 24°C) was added EtOH (1000 mL) under stirring at room temperature in a period of 2h. The resulting mixture was stirred for 1h and filtrated. The precipitate was washed with EtOH (500 mL), filtrated and dried at 25°C/3 mbar to give FS.Na (41 g, 44%).

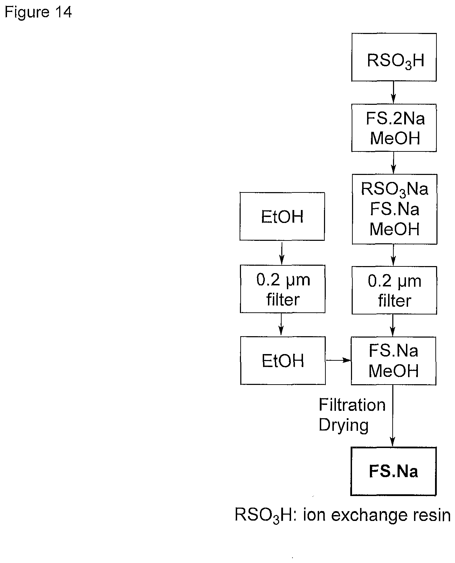

The ion exchange and crystallization process using FS.2Na is depicted in Fig. 14.

### Example 3:

**Ion exchange and crystallization process using FS.THAM [XRPD: FS.Na-form 1]:** To a suspension of ion exchange resin (Dowex Monosphere 650C, 450 g) in MeOH (500 mL) was added FS.THAM (50 g) under stirring at 0°C. The resulting mixture was stirred at 0°C for 1.5h and filtrated (0.2 µm). The resin was washed with cold (0°C) MeOH (2 x 50 mL). To the total filtrate was added a solution of MeONa in MeOH (25 weight-%, 44.0 mL / 41.6 g) under stirring at 0°C. The resulting solution of FS.Na in MeOH was reduced to ca. 80g under vacuum, to which was added EtOH (300 mL) at 0°C dropwise. The resulting suspension was filtrated to give FS.Na (15.3 g, 50%) after drying at 25°C/3 mbar.

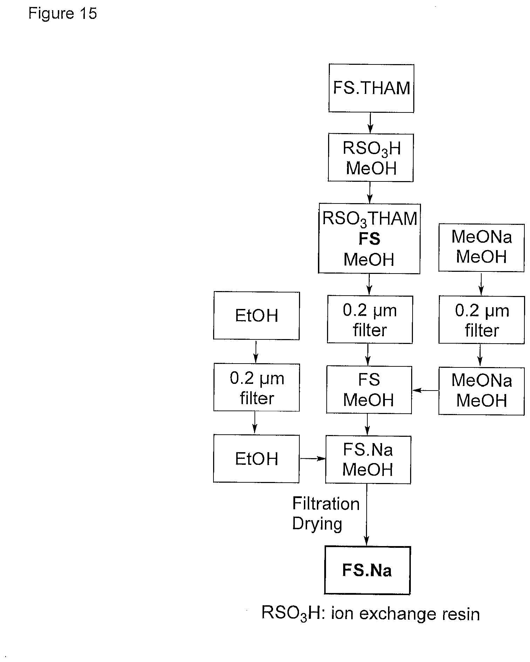

The ion exchange and crystallization process using FS.THAM is depicted in Fig. 15.

### Example 4:

**Acidification with H₂SO₄ in MeOH and crystallization process using FS.2Na [XRPD: FS.Na-form 2]:** To a suspension of FS.2Na (63.6 g) in MeOH (95 mL) was added a solution of H₂SO₄ (95-97%, 9.46 mL) in MeOH (82 mL) under stirring with temperature (< 23°C) and pH (∼5.7) control. The resulting suspension was stirred at room temperature for 1h and filtered under reduced pressure. To the filtrate was added EtOH (300 mL) in a period of 2h at room temperature. The resulting suspension was stirred at room temperature for 1h and at 0°C for 1h. After filtration under reduced pressure the crystal was washed with EtOH (5 x 50 mL), filtrated and dried in fluid bed dryer (N₂, 35°C) to give FS.Na.

### Example 5

**Ion exchange and crystallization process using FS.THAM [XRPD: FS.Na-form 2]:** To a suspension of FS.2Na (106 g) in MeOH (500 mL) was added ion exchange resin (Dowex Monosphere 650C, 250 g, washed with 2 x 300 mL MeOH) under stirring with temperature (< 25°C) and pH (∼6.1) control. The mixture was stirred at room temperature for equilibrium of pH (∼6.1) and then filtrated. To the filtrate (pH 6.1 at 24°C) was added EtOH (1000 mL) under stirring at room temperature in a period of 2h. The resulting mixture was stirred for 1h and filtrated. The crystal was washed with EtOH (2 x 500 mL), filtrated and dried at 25°C/3 mbar to give FS.Na (44 g, 47%).

### Example 6

**Acidification with CF₃CO₂H and crystallization process using FS.2Na [XRPD: FS.Na-form 3]:** To a suspension of FS.2Na (10.0 g) in MeOH (50 mL) was added trifluoroacetic acid (CF₃CO₂H, 2.08 mL) under stirring with temperature (< 23°C) and pH (∼6.6) control. To the resulting solution was added EtOH (500 mL) under stirring at room temperature in a period of 3h. The resulting suspension was stirred at room temperature for 1h and at 0°C for 2h. After filtration under reduced pressure the crystal was dried at 40°C/3 mbar to give FS.Na.

### Example 7:

**Acidification with HCO₂H and crystallization process using FS.2Na [XRPD: FS.Na-form 4]:**To a suspension of FS.2Na (10.0 g) in MeOH (50 mL) was added formic acid (HCO₂H, 2.08 mL) under stirring with temperature (< 23°C) and pH (∼6.6) control. To the resulting solution was added EtOH (500 mL) under stirring at room temperature in a period of 3h. The resulting suspension was stirred at room temperature for 1h and at 0°C for 2h. After filtration under reduced pressure the crystal was dried at 40°C/3 mbar to give FS.Na.

### Example 8:

**Ion exchange and crystallization process using FS.THAM [XRPD: FS.Na-form 5]:** To a suspension of ion exchange resin (Dowex Monosphere 650C, 140 g) in MeOH/EtOH (40/110 mL) was added FS.THAM (15.0 g) under stirring at 0°C in a period of 30 min. The resulting mixture was stirred at 0°C for 1h and filtrated. To the filtrate was added a solution of MeONa in MeOH (25 w%, 13.2 mL / 12.5 g) under stirring at 0°C. The resulting FS.Na in MeOH/EtOH was treated with Molecular Sieve (0.4 nm, 2 mm / 10 mesh) under stirring for 10 min and filtrated. The solvent was removed under vacuum at 40°C to give FS.Na.

### Example 9: Determination of XRPD diffractograms

Powder X-ray diffraction was performed with a PANalytical X'Pert PRO diffractometer equipped with a theta/theta coupled goniometer in transmission geometry, Cu-Kalpha_{1,2} radiation (wavelength 0.15419 nm) with a focusing mirror and a solid state PIXcel detector. Diffractograms (patterns) were recorded at a tube voltage of 45 kV and a tube current of 40 mA, applying a stepsize of 0.013° 2-theta with 40 seconds per step (255 channels) in the angular range of 2° to 40° 2-theta at ambient conditions. A typical precision of the 2-theta values is in the range of ± 0.2° 2-theta, preferably of ± 0.1° 2-theta. Thus, the diffraction peak of a crystalline fosfomycin salt that appears for example at 7.5° 2-theta can appear in the range of from 7.3 to 7.7° 2-theta, preferably in the range of from 7.4 to 7.6 ° 2-theta on most X-ray diffractometers under standard conditions. Measurement was at room temperature and at a relative humidity of 30 %.

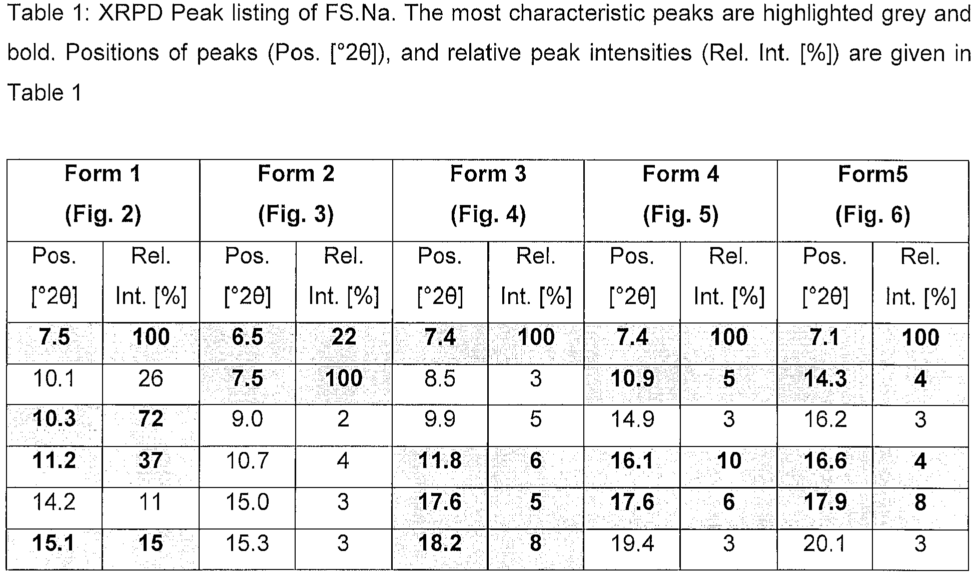

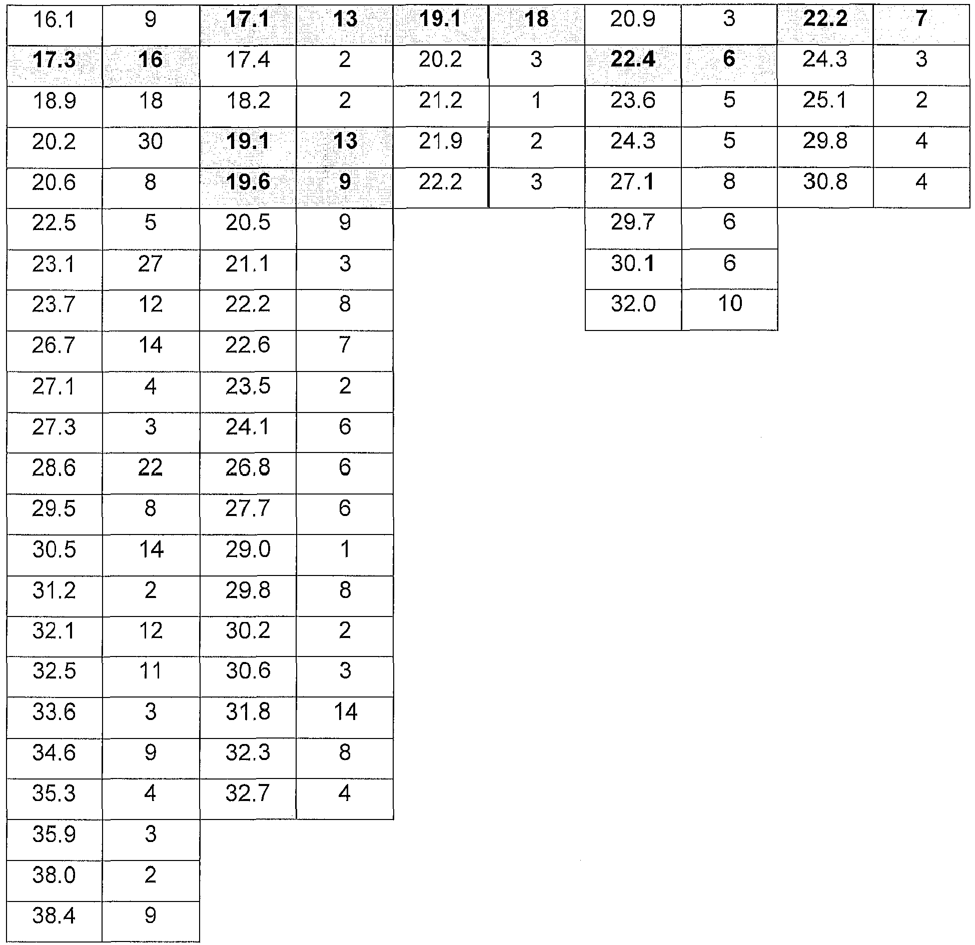

The XRPD patterns of FS.Na-form 1 to 5 show in Figure 2 to 6 respectively.

### Example 10: Determination of bulk and tapped density

The bulk and tapped density was determined using the Tapped Density Tester SVM 121 from ERWEKA, equipped with a 10 mL glass cylinder. Approximately 2 g of the powder was carefully poured into the 10 mL measuring cylinder with a funnel. Bulk density was calculated by dividing the amount of powder (g) by the measured volume (mL). The tapped density was then determined as follows:
- Tap 10 x → Read volume (= V₁₀)
- Tap 500 x (10 + 490) → Read volume (= V₅₀₀)
- Tap 1250 x (500 + 750) → Read volume (= V₁₂₅₀)

(If difference between V₅₀₀ und V₁₂₅₀ is bigger than 0.2 mL (2%), tap another 1250 x and repeat until difference is below 0.2 mL). Tapped density = weight/ volume after tapping. As a measure of flowability, the Hausner factor and Carr Index are calculated from bulk and tapped densities as described below.

### Calculation:

Bulk density = m (mg) / V (mL); Tapped density = m (mg) / V₁₂₅₀ (mL); Hausner-Factor = Tapped density / Bulk density; Carr-Index = [(Tapped density - Bulk density) / Tapped density] * 100%

| **Hausner Factor** | **Compressibility (Carr-Index)** | **Flowability** |
|---|---|---|
| 1.00 - 1.11 | < 10 | Excellent |
| 1.12 - 1.18 | 11 - 15 | Good |
| 1.19 - 1.25 | 16 - 20 | Fair |
| 1.26 - 1.34 | 21 - 25 | Passable |
| 1.35 - 1.45 | 26 - 31 | Bad/cohesive |
| 1.46 - 1.59 | 32 - 37 | Very bad/ very cohesive |
| >1.60 | > 38 | Not flowable at all |

The results are show in Table 2. The final tapped density and tapped volume as well as the calculated compressibility index and Hausner ratio are indicated.

**Table 1 Bulk and Tapped density**

| **Polymorph** | **Bulk density [g/mL]** | **Tapped density [g/mL]** | **Hausner Ratio** | **Carr index [%]** | **Flowability** |
|---|---|---|---|---|---|
| **Form 1** | 0.279 | 0.352 | 1.26 | 21 | Passable |
| **Form 2** | 0.238 | 0.294 | 1.23 | 19 | Fair |
| **FS.2Na** | 0.468 | 0.533 | 1.14 | 10 | Excellent |
| **FS.2Na/SA** | 0.509 | 0.703 | 1.38 | 27 | Bad/cohesive |

### Example 11: Gravimetric Moisture Sorption Measurement

Moisture sorption isotherms were recorded with a SPSx-1µ moisture sorption analyzer (ProUmid, Ulm).
Measurements were started at 3% relative humidity (RH) and then decreased to 0% RH. Afterwards RH was increased from 0% to 90% RH in 5% steps.
The time per step was set to a minimum of 2 hours and a maximum of 6 hours. If an equilibrium condition with a constant mass of ± 0.01% within 1 hour was reached before the maximum time for all examined samples the sequential humidity step was applied before the maximum time of 6 hours. If no equilibrium was achieved the consecutive humidity step was applied after the maximum time of 6 hours. The temperature was 25 ± 0.1 °C.
The displayed figures show the equilibrium points of the sorption curve from 0 - 90% RH, which were recorded at the end of each RH step. The sample weight at 0% RH was used as reference weight.

### a) FS.Na-form 1

The FS.Na-form 1 sample up to 40% r.h. started to absorb water. From 45% r.h. the sample showed clumping and from 55% r.h. liquefied. At the end of the measurement, a liquid residue remained from which no diffractogram could be recorded (cf. Figure 9).

### b) FS.Na-form 2

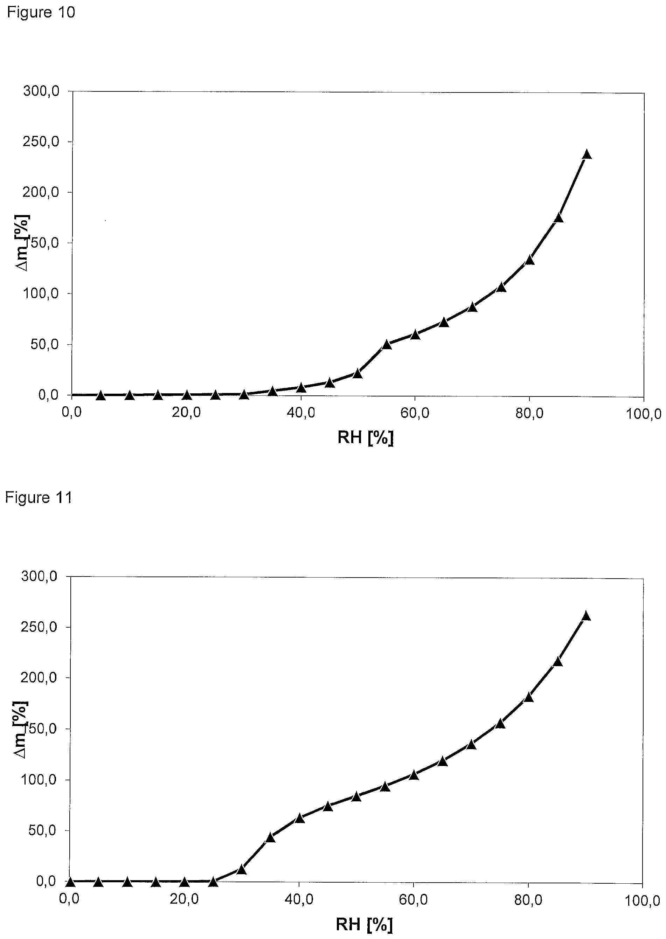

The FS.Na-form 2 sample up to 35% r.h. started to absorb water. From 40% r.h. the sample showed clumping and from 50% r.h. liquefied. At the end of the measurement, a liquid residue remained from which no diffractogram could be recorded (cf. Figure10).

### c) FS.2Na

The FS.2Na sample up to 30% r.h. showed water absorption. From 35% r.h. the sample liquefied. At the end of the measurement, a liquid residue remained from which no diffractogram could be recorded (cf. Figure 11).

### d) FS.2Na/SA by Astro Pharma

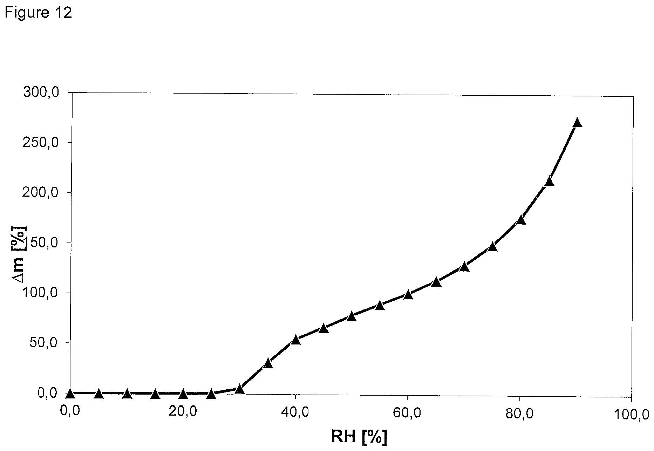

The FS.2Na sample up to 25% r.h. initially showed slight water absorption. From 30% r.h. the water absorption became stronger and the sample liquefied. At the end of the measurement, a liquid residue remained from which no diffractogram could be recorded (cf. Figure 12).

### e) Conclusion

The measurement indicated that FS.2Na and FS.2Na/SA by Astro Pharma liquefied in the range of 30 to 35% r.h., whereas FS.Na-form 1 is stable up to 45% r.h. and FS.Na-form 2 up to 40% r.h.. Therefore, FS.Na-form 1 and 2 have superior moisture sorption properties than FS.2Na and FA.2Na/SA. Moreover, in comparison to FS.Na-form 2, FS.Na-form 1 showed less water uptake at 45% r.h..

### Example 12: Formulations of FS.Na with Arg and Lys

To FS.Na / Arg / Lys or FS.Na / Arg or FS.Na / Lys as a powder mixture were added water to a total volume of 200 mL. The mixture was shaken with hand. Then the pH value and milli-osmolarity (mOsm) of the resulting solution were measured. The solutions of the FS.Na/Arg/Lys and the FS.Na/Lys formulations and one of the FS/Arg formulations were stable at room temperature and 4°C over a period of 24h (stable = no precipitation). Precipitation was determined by visual inspection of the solution after 24h at RT and at 4°C.

**Table 3 Formulation Examples of FS.Na/Arg/Lys and FS.Na/Arg**

| Formulation | FS.Na (g) (mmol) | Arg (g) (mmol) | Lys (g) (mmol) | FS.Na/Arg/Lys (molar ratio) | pH mOsm | Precipitation problem |
|---|---|---|---|---|---|---|
| 1 | 9.28 | --- | --- | --- | ∼ 5.0 | no |
| | 57.98 | | | | n.d. | |
| 2 | 9.28 | 3.0 | 2.4 | 1.0 : 0.30 : 0.28 | 6.6 | no |
| | 57.98 | 17.22 | 16.42 | | 630 | |
| 3 | 9.28 | 4.5 | 1.3 | 1.0 : 0.45 : 0.15 | 6.5 | no |
| | 57.98 | 25.83 | 8.89 | | 640 | |
| 4 | 9.28 | 2.0 | 1.3 | 1.0 : 0.20 : 0.15 | 6.3 | no |
| | 57.98 | 11.48 | 8.89 | | 597 | |
| 5 | 9.28 | 4.0 | 2.4 | 1.0 : 0.40 : 0.28 | 6.9 | no |
| | 57.98 | 22.96 | 16.42 | | n.d. | |
| 6 | 9.28 | 2.0 | --- | 1.0 : 0.20 | 5.9 | yes |
| | 57.98 | 11.48 | | | 575 | |
| 7 | 9.28 | | --- | 1.0 : 0.30 | 6.2 | yes |
| | 57.98 | 3.0 17.22 | | | 544 | |
| 8 | 9.28 | 5.0 | --- | 1.0 : 0.50 | 6.5 | yes |
| | 57.98 | 28.70 | | | 627 | |
| 9 | 9.28 | 6.0 | | 1.0 : 059 | 6.7 | yes |
| | 57.98 | 34.44 | --- | | 636 | |
| 10 | 9.28 | 8.0 | --- | 1.0 : 0.79 | 7.4 | no |
| | 57.98 | 45.92 | | | 656 | |
| 11 | 9.28 | 9.0 | | 1.0:0.89 | 7.6 | no |
| | 57.98 | 51.66 | --- | | 670 | |
| 12 | 9.28 | 10.0 | --- | 1.0 : 0.99 | 8.4 | no |
| | 57.98 | 57.44 | | | 702 | |
| 13 | 9.28 | --- | 3.0 | 1.0 : 0.35 | | no |
| | 57.98 | | 20.52 | | 620 | |
| 14 | 9.28 | --- | 5.0 | 1.0 : 0.59 | 6.6 | no |
| | 57.98 | | 34.20 | | 658 | |
| 15 | 9.28 | --- | 6.0 | 1.0 : 0.71 | 6.9 | no |
| | 57.98 | | 41.04 | | 676 | |
| 16 | 9.28 | --- | 7.0 | 1.0 : 0.83 | 7.3 | no |
| | 57.98 | | 47.88 | | 694 | |
| 17 | 9.28 | --- | 8.0 | 1.0 : 0.94 | 8.0 | no |
| | 57.98 | | 54.72 | | 719 | |

### Example 13: Preparation of Formulations of FS.Na with Arg and Lys

Formulations such as the exemplary formulations disclosed in Example 12 and in Table 3 can be prepared by various method for example by the methods (a), (b) or (c) as described below:
(a) Solid FS.Na and solid arginine and/or lysine can be mixed as a powder into a single container, for example resulting in a molar ratio of a final solid product as disclosed in Table 3.
(b) Solid FS.Na can be placed in a first container and in a second container a stable solution of arginine and/or lysine in a pharmaceutically acceptable solvent such as water is provided. An instruction leaflet explains to the user of the product, how to use the arginine and/or lysine solution of the second container to dissolve the solid FS.Na present in the first container. Dissolving the contents of the first container using the liquid of the second container results for example in a molar ratio of the final product as disclosed in Table 3.
(c) In a first step a liquid solution of FS.Na and a liquid solution of arginine and/or lysine are prepared using a pharmaceutically acceptable solvent such as water. If both arginine and lysine are desired to be present in the final product it is possible to prepare one solution containing arginine and lysine within the same solution or it is possible to prepare a separate arginine solution and a separate lysine solution.

In a second step the solutions are combined.
In a third step the solvent of the combined solution of step 2 is removed for example by commonly known methods such as freeze-drying, lyophilization, etc. under conditions such as temperature and pressure, which ensure that FS.Na and arginine and/or lysine are not subject to degradation. The final solid product for example is obtained in a molar ratio as disclosed in Table 3.

Preferably for all three methods (a) to (c) as starting material sterile FS.Na, sterile arginine and/or sterile lysine is used. In methods (b) or (c) the liquid materials alternatively might be sterilized by for example sterile filtration. Alternatively in method (c), step 2, the liquid mix of FS.Na and arginine and/or lysine can be sterilized by sterile filtration. Furthermore for all three methods (a) to (c) the solid starting materials or the solid final product comprising FS.Na and/or arginine and/or lysine can be sterilized by radiation, for example by gamma-radiation. The final product alternatively can be sterilize by radiation such as gamma-radiation of the finally packaged solid dose form.

## Claims

1. Crystalline fosfomycin salt comprising 0.8 to 1.2 equivalents sodium relative to one equivalent fosfomycin.

2. Crystalline fosfomycin salt according to claim 1, which is fosfomycin monosodium (FS.Na).

3. Crystalline fosfomycin salt according to claims 1 or 2, wherein
(i) the fosfomycin monosodium (FS.Na) is **characterized by** an XRPD pattern as measured using Cu-Kα1.2 radiation at a wavelength of 0.15419 nm comprising peaks at 2-theta angles (°2θ) of 7.5 ± 0.2, 10.3 ± 0.2 and 11.2 ± 0.2 ("**Form 1**"); or
(ii) the fosfomycin monosodium (FS.Na) is **characterized by** an XRPD pattern as measured using Cu-Kα1.2 radiation at a wavelength of 0.15419 nm comprising peaks at 2-theta angles (°2θ) of 6.5 ± 0.2, 7.5 ± 0.2 and 17.1 ± 0.2 ("**Form 2**"); or
(iii) the fosfomycin monosodium (FS.Na) is **characterized by** an XRPD pattern as measured using Cu-Kα1.2 radiation at a wavelength of 0.15419 nm comprising peaks at 2-theta angles (°2θ) of 7.4 ± 0.2, 11.8 ± 0.2 and 18.2 ± 0.2 ("**Form 3**"); or
(iv) the fosfomycin monosodium (FS.Na) is **characterized by** an XRPD pattern as measured using Cu-Kα1.2 radiation at a wavelength of 0.15419 nm comprising peaks at 2-theta angles (°2θ) of 7.4 ± 0.2, 10.9 ± 0.2 and 17.6 ± 0.2 ("**Form 4**"); or
(v) the fosfomycin monosodium (FS.Na) is **characterized by** an XRPD pattern as measured using Cu-Kα1.2 radiation at a wavelength of 0.15419 nm comprising peaks at 2-theta angles (°2θ) of 7.1 ± 0.2, 16.6 ± 0.2 and 17.9 ± 0.2 ("**Form 5**").

4. Pharmaceutical composition comprising the crystalline fosfomycin salt according to any one of claims 1 to 3.

5. Pharmaceutical composition according to claim 4, additionally comprising arginine and/or lysine, wherein the respective amounts of arginine and/or lysine being present in the pharmaceutical composition are such that the pH of the pharmaceutical composition, if reconstituted in water, is in a physiological range of 6.0 to 8.0, and no precipitate is visible after visual examination.

6. Pharmaceutical composition according to claim 4 or 5, wherein the molar ratio of the amount of FS.Na : Arg is in a range of about 1.0 : 0.7-0.95; preferably 1.0 : 0.7, more preferably about 1.0 : 0.9, and most preferably about 1.0 : 0.8, or wherein the molar ratio of the amount of FS.Na : Lys is in a range of about 1.0 : 0.35-0.95; preferably 1.0 : 0.9, of about 1.0 : 0.8 , of about 1.0 : 0.7, more preferably about 1.0 : 0.5, and most preferably about 1.0 : 0.6.

7. Pharmaceutical composition according to claim 4 or 5, wherein the composition comprises FS.Na, Arginine and Lysine, preferably in a molar ratio of 1.0 : 0.1-0.5 : 0.1-0.4; preferably 1.0 : 0.2 : 0.15; more preferably in a molar ratio of 1.0 : 0.45 : 0.15; and most preferably in a molar ratio of 1.0 : 0.3 : 0.3.

8. Pharmaceutical composition according to any one of claims 4 to 7, being a solid preparation, preferably a powder, or being a kit comprising one container containing solid FS.Na and another container containing a liquid comprising arginine and/or lysine, wherein said liquid preferably is an aqueous liquid.

9. Injectable preparation comprising a solution of FS.Na, arginine and/or lysine in water, wherein said injectable preparation has a pH in a physiological range of 6.0 to 8.0 and is ready to use, preferably wherein no precipitate is visible after visual examination, for example after eye examination.

10. Crystalline fosfomycin salt according to any one of claims 1 to 3, pharmaceutical composition according to any one of claims 4 to 7, or injectable preparation according to claim 8, for parenteral use in therapy.

11. Crystalline fosfomycin salt, pharmaceutical composition, or injectable preparation according to claim 10, for use in treating of a patient having a bacterial infection.

12. Crystalline fosfomycin salt, pharmaceutical composition, or injectable preparation for use according to claim 11, wherein the bacterial infection is a genitourinary disease, in particular urinary tract infection.

13. Crystalline fosfomycin salt, pharmaceutical composition, or injectable preparation for use according to claim 11 or 12, wherein said patient having a bacterial infection in addition suffers from a condition for which limitation of uptake of sodium is clinically indicated.

14. Crystalline fosfomycin salt, pharmaceutical composition, or injectable preparation for use according to claim 13, wherein said condition is selected from the group consisting of cardiac insufficiency, hypertension, pulmonary oedema and diabetes.

15. Use of crystalline fosfomycin salt according to any one of claims 1 to 3, or of pharmaceutical composition according to any one of claims 4 to 8, for the preparation of an injectable preparation according to claim 9.
